# EUROPEAN PATENT SPECIFICATION

(11) **EP 2 201 888 B1**
(45) Date of publication and mention of the grant of the patent: **14.12.2016**
(21) Application number: 08840628.5
(22) Date of filing: 16.09.2008
(51) Int. Cl.: A61B 1/00, A61B 1/04

(54) **ENDOSCOPIC APPARATUS AND SETTING METHOD THEREOF**
ENDOSKOPISCHE VORRICHTUNG UND EINSTELLVERFAHREN DAFÜR
APPAREIL ENDOSCOPIQUE ET SON PROCÉDÉ DE RÉGLAGE

(30) Priority: 18.10.2007 JP 2007271665
(43) Date of publication of application: 30.06.2010
(73) Proprietor: Olympus Corporation, Tokyo 192-8507 (JP)
(72) Inventor: TAKEI, Shunji, Shibuya-ku Tokyo 151-0072 (JP)
(74) Representative: Gunzelmann, Rainer
(86) International application number: PCT/JP2008/066672
(87) International publication number: WO 2009/050972

(56) References cited:
- EP-A1- 1 491 133
- DE-A1-102006 049 856
- JP-A- 2001 029 313
- JP-A- 2003 079 570
- JP-A- 2005 013 279
- JP-A- 2005 087 450

## Description

### Technical Field

The present invention relates to an endoscope apparatus for obtaining a reflected light image and a fluorescence image and a method of setting the same.

### Background Art

In recent years, endoscopes have been widely used in fields of medical care and industry. Particularly in the field of medical care, a technique of obtaining an image has been proposed in which a normal tissue and a lesion tissue can be easily identified, in addition to an endoscope apparatus for obtaining a normal image with normal white light.

For example, Japanese Patent Application Laid-Open Publication No. 2001-137174 discloses an apparatus which generates a display signal mainly by reflecting a relative intensity of fluorescence to a color and reflecting an intensity of reference light to a luminance. Further, Japanese Patent Application Laid-Open Publication No. 2000-270265 discloses an apparatus for superimposing a fluorescence image on a background image.

In these techniques, however, an intensity of fluorescence emitted from a normal tissue varies among patients. Thus colors of normal tissues may vary among patients and it may be difficult to identify a lesion tissue and a normal tissue. Further, light reflected in a wide band may degrade a function of obtaining an image on which a normal tissue and a lesion tissue can be identified with sufficient ease.

To address this problem, for example, Japanese Patent Application Laid-Open Publication No. 2003-126014 proposes an endoscope apparatus which can obtain an image on which a normal tissue and a lesion tissue can be easily identified.

For example, in an endoscope apparatus capable of simultaneously observing normal light and fluorescence as disclosed in Japanese Patent Application Laid-Open Publication No. 2003-126014, however, proper brightness may not be obtained on a fluorescence image (the image may become too bright or too dark) when the apparatus adjusts, for example, an amount of light to obtain proper brightness on a normal light image, so that a desired image cannot be obtained.

EP 1 491 133 A1 may be construed to disclose an endoscope apparatus comprising an endoscope having an image pickup element having a sensitivity, which can be changed by multiplying generated charges by supplied pulse-type signals, light source means for irradiating light to an object, aperture means for adjusting an amount of light to be irradiated to the object, driver means for adjusting and supplying the pulse-type signals to the image pickup element in order to change the sensitivity of the image pickup element, metering means for generating an intensity signal based on a signal output from the image pickup element, sensitivity control means for, based on the intensity signal of the metering means, supplying to the driver means a sensitivity control signal for generating the pulse-type signals for controlling a charge multiplication factor of the image pickup element, metering correcting means for correcting the intensity signal by the metering means based on the charge multiplication factor, and aperture control means for controlling the aperture means based on a metering signal corrected by the metering correcting means.

Moreover, in order to adjust both of a normal light image and a fluorescence image to proper brightness, it is necessary to provide different light sources for respective observation modes. In this case, the apparatus may be disadvantageously increased in size.

The present invention has been devised in view of the foregoing circumstances. An object of the present invention is to provide an endoscope apparatus which can adjust a normal light image and a fluorescence image to proper brightness with a simple and inexpensive configuration.

### Disclosure of Invention

### Means for Solving the Problem

There are provided an apparatus and a method of the independent claims. Developments are seen in the depedent claims.

Preferably, an endoscope apparatus includes: an endoscope having a first image pickup unit for normal light observation which irradiates a subject with illumination light to pick up an image of reflected light from the subject and a second image pickup unit for fluorescence observation which irradiates the subject with excitation light to pick up an image of fluorescence from the subject; a light source section for emitting the illumination light and the excitation light; a dimming signal generating section for generating a dimming signal based on image pickup signals picked up by the first image pickup unit and the second image pickup unit; a light source control section for controlling the light source section; a signal amplification section for amplifying signal intensities of the image pickup signals picked up by the first image pickup unit and the second image pickup unit; an electronic shutter control section for controlling an electronic shutter which stores charge and discharges the charge in the first image pickup unit and/or the second image pickup unit; a comparison section for comparing the dimming signal with a predetermined reference level signal; and a setting section for setting an amount of light emitted from the light source section controlled by the light source control section, an amplification factor in the signal amplification section corresponding to the first image pickup unit and/or the second image pickup unit, and a charge control parameter in the electronic shutter control section corresponding to the first image pickup unit and/or the second image pickup unit, based on a comparison result of the comparison section.

Preferably, an endoscope apparatus includes: an endoscope having an image pickup unit for irradiating a subject with illumination light to pick up a normal light observation image of reflected light from the subject and irradiating the subject with excitation light to pick up a fluorescence observation image of fluorescence from the subject; a light source section for emitting the illumination light and the excitation light; a dimming signal generating section for generating a dimming signal based on an image pickup signal picked up by the image pickup unit; a light source control section for controlling the light source section; a signal amplification section for amplifying a signal intensity of the image pickup signal picked up by the image pickup unit; an electronic shutter control section for controlling an electronic shutter which stores charge and discharges the charge in the image pickup unit; a comparison section for comparing the dimming signal with a predetermined reference level signal; and a setting section for setting an amount of light emitted from the light source section controlled by the light source control section, an amplification factor in the signal amplification section corresponding to the normal light observation image and/or the fluorescence observation image, and a charge control parameter in the electronic shutter control section corresponding to the normal light observation image and/or the fluorescence observation image, based on a comparison result of the comparison section.

Preferably, a method of setting an endoscope apparatus the endoscope apparatus having an image pickup unit for irradiating a subject with illumination light to pick up a normal light observation image of reflected light from the subject and irradiating the subject with excitation light to pick up a fluorescence observation image of fluorescence from the subject, the method including the steps of: generating, by a dimming signal generating section, a dimming signal based on an image pickup signal picked by the image pickup unit; comparing, by a comparison section, the dimming signal with a predetermined reference level; and setting, by a setting section, amounts of the illumination light and the excitation light, an amplification factor of the image pickup signal corresponding to the normal light observation image and/or the fluorescence observation image, and a charge control parameter corresponding to the normal light observation image and/or the fluorescence observation image, based on a comparison result of the comparison section.

### Brief Description of the Drawings

Fig. 1 is a configuration diagram showing a configuration of an endoscope apparatus according to an embodiment of the present invention;
Fig. 2 is a view showing a configuration of a switching filter shown in Fig. 1;
Fig. 3 is a timing chart for explaining a fluorescence image and a normal image that are obtained by the endoscope apparatus shown in Fig. 1;
Fig. 4 is a flowchart for explaining processing of a processor shown in Fig. 1;
Fig. 5 is a flowchart for explaining a modification of processing of Fig. 3;
Fig. 6 is a view showing an encapsulated endoscope which is a first modification of an electronic endoscope shown in Fig. 1; and
Fig. 7 is a view showing a second modification of the electronic endoscope shown in Fig. 1.

### Best Mode for Carrying Out the Invention

The following will describe an embodiment of the present invention with reference to accompanying drawings.

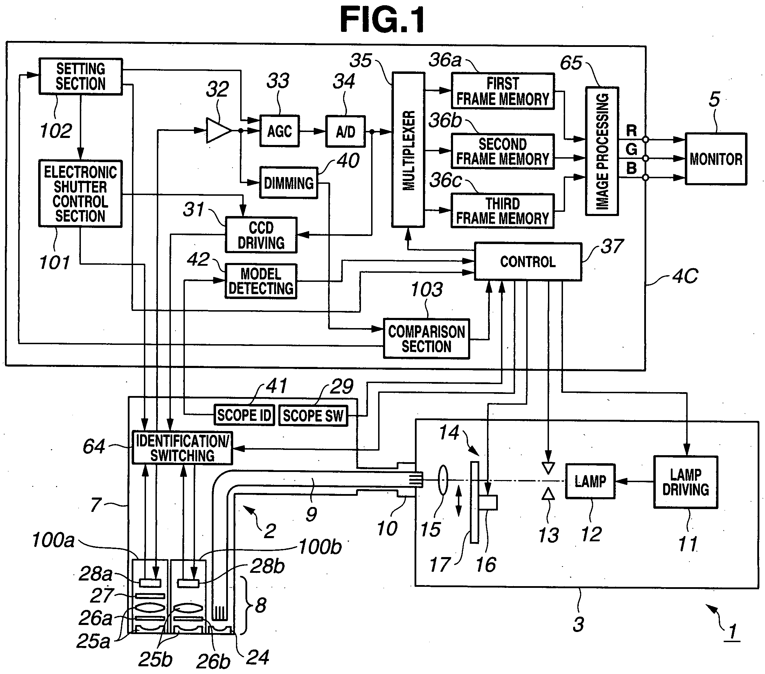

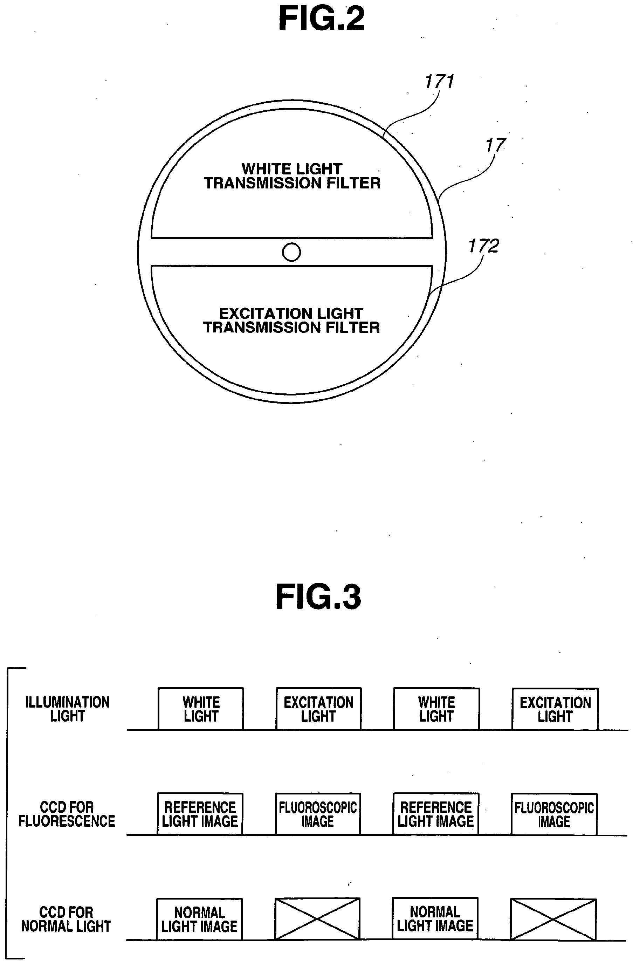

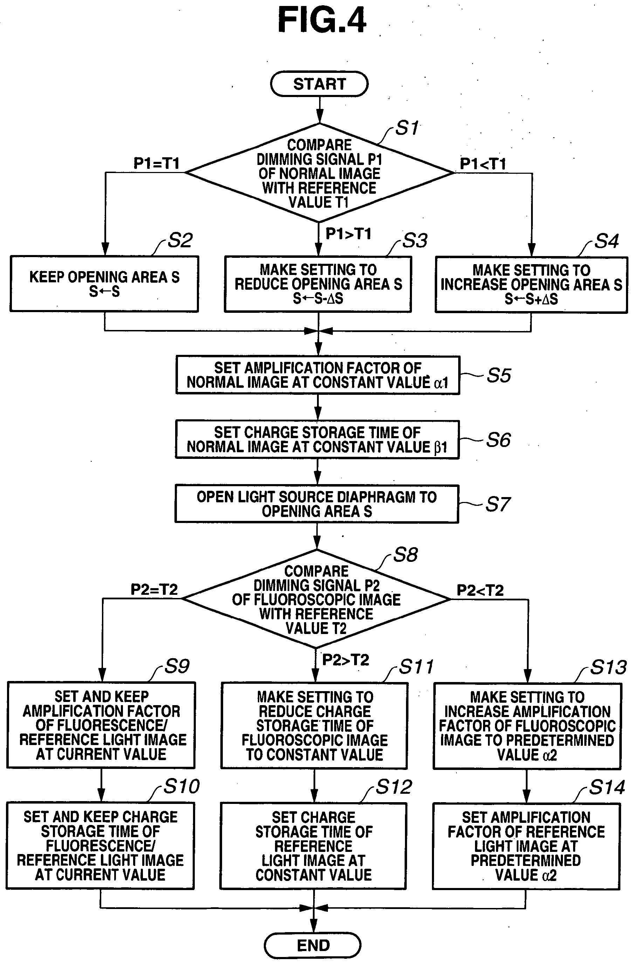

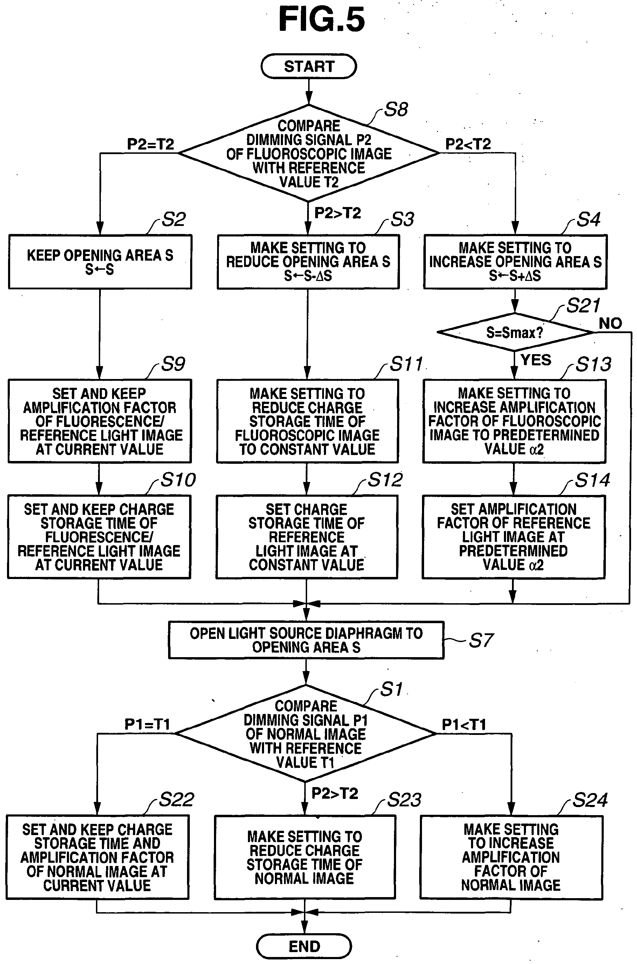

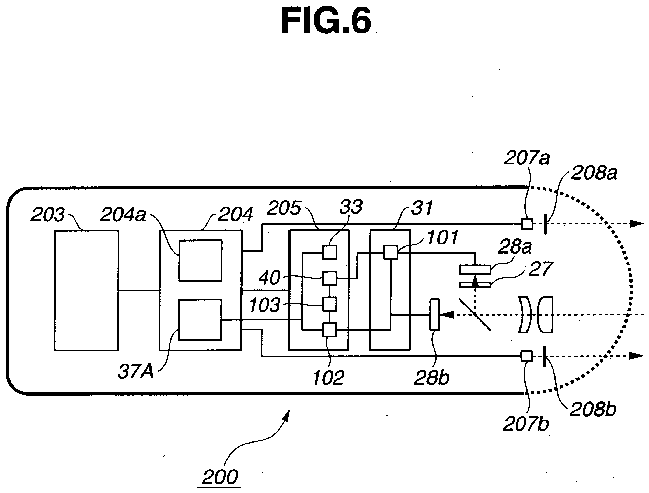

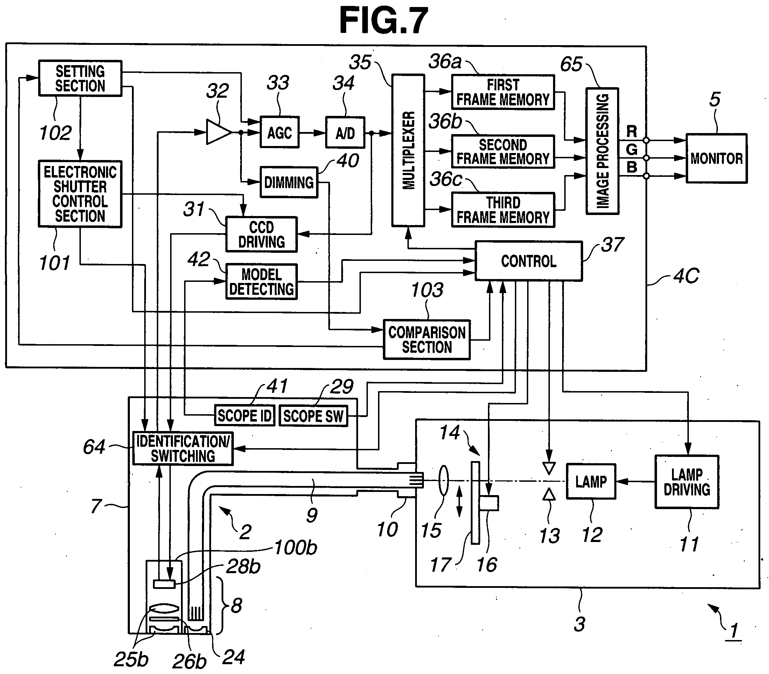

Figs. 1 to 6 relate to the embodiment of the present invention. Fig. 1 is a configuration diagram showing a configuration of an endoscope apparatus. Fig. 2 is a view showing a configuration of a switching filter shown in Fig. 1. Fig. 3 is a timing chart for explaining a fluorescence image and a normal image that are obtained by the endoscope apparatus shown in Fig. 1. Fig. 4 is a flowchart for explaining processing of a processor shown in Fig. 1. Fig. 5 is a flowchart for explaining a modification of processing of Fig. 3. Fig. 6 is a view showing an encapsulated endoscope which is a first modification of an electronic endoscope shown in Fig. 1. Fig. 7 is a view showing a second modification of the electronic endoscope shown in Fig. 1.

### (Configuration)

As shown in Fig. 1, an endoscope apparatus 1 having normal observation mode and fluorescence observation mode according to the present embodiment is made up of an electronic endoscope 2 which is inserted for observation into a body cavity, a light source device 3 for generating normal light, which is white light, and excitation light for exciting autofluorescence (hereinafter, will be simply referred to as fluorescence), a processor 4 which performs signal processing for constructing a normal observation image (hereinafter, will be simply referred to as a normal image) formed by normal light and a fluorescence image formed by fluorescence, and a monitor 5 for displaying a normal image and a fluorescence image.

The electronic endoscope 2 has an elongated insertion portion 7 inserted into the body cavity, and a distal end portion 8 of the insertion portion 7 includes illumination means and image pickup means.

In the insertion portion 7, a light guide fiber 9 is inserted to transmit (guide) normal light and excitation light. A light source connector 10 provided on an incidence end on an operator's hand side of the light guide fiber 9 is detachably connected to the light source device 3.

The light source device 3 includes a lamp 12 which is driven by a lamp driving circuit 11 to emit light including light from an infrared wavelength band to a visible light band, a light source diaphragm 13 which is provided on an illumination light path of the lamp 12 and limits an amount of light from the lamp 12, a switching filter portion 14 provided on the illumination light path, and a condenser lens 15 for condensing light having passed through the switching filter portion 14.

The switching filter portion 14 is configured by including a switching filter 17 rotated by a rotation motor 16. As shown in Fig. 2, the switching filter 17 has a white light transmission filter 171 allowing passage of white light for normal observation and an excitation light transmission filter 172 allowing passage of excitation light for fluorescence observation. The white light transmission filter 171 and the excitation light transmission filter 172 are rotationally driven by the rotation motor 16 so as to be sequentially inserted into an optical path substantially in a continuous manner.

Referring to Fig. 1 again, illumination light from the light source device 3 is transmitted (guided) to a distal end side of the insertion portion 7 of the electronic endoscope 2 through the light guide fiber 9. The light guide fiber 9 transmits excitation light and normal light with a small transmission loss. The light guide fiber 9 is composed of, for example, a multi-component glass fiber, a quarts fiber, and so on.

The light transmitted to a dismal end face of the light guide fiber 9 is emitted in an expanded manner to a side of an observed portion (not shown) in the body cavity through an illumination lens 24 attached to an illumination window (not shown) opposed to the distal end face.

In the electronic endoscope 2, a fluorescence observation CCD (CCD for fluorescence) 28a and a normal observation CCD (CCD for normal light) 28b are provided on the distal end portion 8 of the insertion portion 7. The CCDs 28a and 28b are made up of, for example, charge-coupled devices (will be abbreviated to CCDs) serving as image pickup devices. The distal end portion 8 has an observation window (not shown) next to the illumination window (not shown). On the observation window (not shown) of the distal end portion 8, an image pickup section 100a for fluorescence observation is installed as a second image pickup unit and an image pickup section 100b for normal light observation is installed as a first image pickup unit.

The image pickup device for picking up a fluorescence image and a normal image may be a CMD (Charged Modulation Device) image pickup device, a C-MOS image pickup device, an AMI (Amplified MOS imager), and a BCCD (Back Illuminated CCD) instead of the CCDs 28a and 28b.

The image pickup section 100a for fluorescence observation is made up of an objective lens system 25a for forming an optical image, a first diaphragm 26a for limiting an amount of light to spatially adjust a focus from a far point to a near point, an excitation light cut-off filter 27 for cutting off excitation light, and the fluorescence observation CCD 28a serving as an image pickup device for picking up a fluorescence image.

The image pickup section 100b for normal observation is made up of an objective lens system 25b for forming an optical image, a second diaphragm 26b, and the normal observation CCD 28b serving as an image pickup device for picking up a normal image. In this configuration, the normal observation CCD 28b has a color filter (not shown) on an image pickup surface and outputs an optical image as a spectral image having been divided into RGB.

The first diaphragm 26a has an fNo. smaller than an fNo. of the second diaphragm 26b. In other words, a larger amount of light is incident on the CCD 28a for fluorescence.

Further, the excitation light cut-off filter 27 is a filter for cutting off light excited for generating fluorescence during a fluorescence observation. For example, the excitation light cut-off filter 27 has a property of allowing passage of a wavelength band of 470 nm to 700 nm, that is, allowing passage of visible light other than some wavelengths (400 nm to 470 nm) of a blue band.

As shown in Fig. 3, a living tissue is irradiated with substantially continuous illumination light of white light and excitation light supplied from the light source device 3. In fluorescence image mode, the fluorescence observation CCD 28a substantially continuously picks up an image of reference light (reference light image) reflected from the living tissue through the excitation light cut-off filter 27 during irradiation of white light and an image of (auto) fluorescence (fluorescence image) excited from the living tissue during irradiation of excitation light.
Moreover, in normal image mode, the normal observation CCD 28b picks up a normal light image on the living tissue during irradiation of white light.

Referring to Fig. 1 again, the electronic endoscope 2 includes a scope switch 29 for performing instructions for selecting the fluorescence image mode and the normal image mode and instructions for freezing and releasing. An operation signal of the scope switch 29 is inputted to a control circuit 37 serving as light source control means, and the control circuit 37 performs a controlling operation in response to the operation signal.

The two CCDs 28a and 28b are connected to a CCD driving circuit 31, a preamplifier 32, and an electronic shutter control section 101 via a device identification/switching section 64. Switching of the device identification/switching section 64 is controlled by the control circuit 37. In other words, when the fluorescence image mode is selected by the scope switch 29, the CCD 28a for fluorescence is selected and used. When the normal image mode is selected, the CCD 28b for normal light is selected and used.

In the normal image mode, the CCD 28b for normal light is driven by a CCD drive signal from the CCD driving circuit 31 provided in the processor 4, photoelectrically converts an optical image formed on the CCD 28b for normal light, and outputs an image signal.

In the fluorescence image mode, the fluorescence observation CCD 28a is driven by the CCD drive signal from the CCD driving circuit 31 provided in the processor 4, photoelectrically converts an optical image formed on the fluorescence observation CCD 28a, and outputs an image signal.

The image signal is amplified by the preamplifier 32 provided in the processor 4, is further amplified to a predetermined level by an automatic gain control (AGC) circuit 33 serving as signal amplifying means, and then is converted from an analog signal to a digital signal (image data) by an A/D converter circuit 34. Respective pieces of image data are temporarily stored (retained) in a first frame memory 36a, a second frame memory 36b, and a third frame memory 36c through a multiplexer 35 for switching inputs and outputs.

The control circuit 37 controls switching of the multiplexer 35 such that in the normal image mode, respective pieces of image data are sequentially stored in the first frame memory 36a, the second frame memory 36b, and the third frame memory 36c from the spectral image having been divided into RGB through the color filter (not shown) of the normal observation CCD 28b.

Further, the control circuit 37 controls the switching of the multiplexer 35 such that in the fluorescence image mode, image data of the reference light and image data of fluorescence generated from the living tissue by excitation light are sequentially stored in, for example, the first frame memory 36a and the second frame memory 36b, respectively. The reference light is reflected light from the living tissue in a narrow band through the excitation light cut-off filter 27 during the irradiation of normal light.

The pieces of image data stored in the frame memories 36a to 36c are inputted to an image processing circuit 65 and are subjected to image processing in which an input signal undergoes, for example, matrix conversion into an output signal with a hue allowing a normal tissue portion and a lesion tissue portion to be easily identified. After that, the image data is converted to an analog RGB signal and is outputted to the monitor 5.

The processor 4 includes a dimmer circuit 40 serving as dimming signal generating means for generating a dimming signal for controlling an opening area of the light source diaphragm 13 in the light source device 3 based on the signal having passed through the preamplifier 32. Moreover, the opening area of the light source diaphragm 13 is controlled by the control circuit 37.

The control circuit 37 further controls a lamp current, which drives emission of the lamp 12, from the lamp driving circuit 11. Moreover, the control circuit 37 performs a controlling operation in response to an operation of the scope switch 29.

The processor 4 includes a comparison section 103 serving as comparison means for comparing an output (dimming signal) of the dimmer circuit 40 with a predetermined reference value, a setting section 102 serving as setting means for setting a gain of the AGC 33, a shutter control parameter (charge storage time) in the electronic shutter control section 101, and the opening area of the light source diaphragm 13 in the light source device 3 based on a comparison result of the comparison section 103, and the electronic shutter control section 101 serving as electronic shutter control means for controlling an electronic shutter function of the CCD driving circuit 31 by control of the setting section 102. The detail of the comparison section 103, the setting section 102, and the electronic shutter control section 101 will be described later. The comparison result of the comparison section 103 is also inputted to the control circuit 37.

The electronic endoscope 2 has a scope ID generating section 41 for generating unique ID information including at least a model of the electronic endoscope 2. When the electronic endoscope 2 is connected to the processor 4, a model detecting circuit 42 provided the processor 4 side detects model information of the connected electronic endoscope 2 and transmits the model information to the control circuit 37.

The control circuit 37 transmits a control signal for properly setting a parameter for matrix processing of an image processing circuit 38, according to characteristics of the model of the connected electronic endoscope 2.

### (Operation)

The following will describe an operation of the present embodiment configured thus. As shown in Fig. 4, in the processor 4, the comparison section 103 compares in step S1 a predetermined reference value T1 and a dimming signal P1 transmitted from the dimmer circuit 40 for a normal light image (see Fig. 3) on the normal observation CCD 28b at the irradiation of white light, and the comparison section 103 outputs a comparison result to the setting section 102.

In the case of P1 = T1, the setting section 102 sets the control circuit 37 in step S2 so as to keep an opening area S of the light source diaphragm 13 in the light source device 3 (S ← S), and then a process advances to step S5.

In the case of P1 > T1, the setting section 102 sets the control circuit 37 in step S3 so as to reduce the opening area S of the light source diaphragm 13 in the light source device 3 by a predetermined amount ΔS (S ← S - ΔS), and then the process advances to step S5.

In the case of P1 < T1, the setting section 102 sets the control circuit 37 in step S4 so as to increase the opening area S of the light source diaphragm 13 in the light source device 3 by the predetermined amount ΔS (S ← S + ΔS), and then the process advances to step S5.

In step S5, the setting section 102 sets an amplification factor (gain) of the AGC 33 at a constant value α1. Further, the setting section 102 sets the electronic shutter control section 101 in step S6 so as to set, at a constant value β1, the charge storage time for controlling charge storage of the electronic shutter function of the CCD driving circuit 31. Next, in step S7, the setting section 102 opens the light source diaphragm 13 in the light source device 3 to the opening area S set in steps S2 to S4.

Next, in step S8, the comparison section 103 compares a predetermined reference value T2 with a dimming signal P2 transmitted from the dimmer circuit 40 for a fluorescence image (see Fig. 3) on the fluorescence CCD 28a at the irradiation of excitation light, and the comparison section 103 outputs a comparison result to the setting section 102.

In the case of P2 = T2, the setting section 102 sets the electronic shutter control section 101 so as to set and keep, at a current value, the amplification factor (gain) of the AGC 33 for the fluorescence image and the reference light image in step S9 and set and keep, at a current value, the charge storage time for the fluorescence image and the reference light image in step S10, and then the processing is completed.

In the case of P2 > T2, the setting section 102 sets the electronic shutter control section 101 in step S11 so as to reduce the charge storage time of the fluorescence image to a constant value, and sets the electronic shutter control section 101 in step S12 so as to set the charge storage time of the reference light image at a constant value, and then the processing is completed.

In the processing of steps S11 and S 12, the amplification factor (gain) of the AGC 33 for the fluorescence image and the reference light image is set and kept at the current value.

In the case of P2 < T2, in step S 13, the setting section 102 increases the amplification factor (gain) of the AGC 33 for the fluorescence image and sets the amplification factor at a constant value α2 (> a current amplification factor). Further, in step S 14, the setting section 102 also sets the amplification factor (gain) of the AGC 33 for the reference light image at the constant value α2 (> the current amplification factor), and then the processing is completed.

For example, in the present embodiment, a series of these steps (steps S1 to S 13) is always repeated during simultaneous observation of normal observation and fluorescence observation.

In the processing of steps S 13 and S 14, for the fluorescence image and the reference light image, the charge storage time for controlling the charge storage of the electronic shutter function of the CCD driving circuit 31 is set and kept at the current value.

Although the predetermined reference values T1 and T2 are determined beforehand, a setting of the reference values can be changed by, for example, a user. Further, the reference values may be changed to values according to a type of the light source device.

As described above, in the present embodiment, (1) in the normal observation mode, the dimming signal from the dimmer circuit 40 is compared with the first reference value T1, and the amplification factor (gain) of the AGC 33, the charge storage time of the electronic shutter function, and the opening area of the light source diaphragm 13 are set based on the comparison result. (2) Further, in the fluorescence observation mode, a set state of the opening area of the light source diaphragm 13 in the normal observation mode is kept, the dimming signal from the dimmer circuit 40 is compared with the second reference value T2, and the amplification factor (gain) of the AGC 33 and the charge storage time of the electronic shutter function are set based on the comparison result. Thus the amplification factor (gain) of the AGC 33 and the charge storage time of the electronic shutter function can be set according to the observation mode with a simple configuration of the comparison section 103 and the setting section 102, and the normal light image and the fluorescence image can be inexpensively adjusted to proper brightness with ease.

Particularly, in the present embodiment, even when a normal image and a fluorescence image are simultaneously obtained using the single light source device and the two image pickup devices, it is possible to achieve an effect of obtaining proper brightness on both images.

### (First Modification)

In the foregoing embodiment, as described above, brightness is adjusted in the normal observation mode by first setting/controlling the opening of the diaphragm, the amplification factor, and the charge storage time, and then the brightness is adjusted in the fluorescence observation mode by setting/controlling the amplification factor and the charge storage time. The present invention is not limited to the above adjustment. The brightness may be adjusted first in the fluorescence observation mode and then adjusted in the normal observation mode.

In the foregoing embodiment, the opening area of the light source diaphragm 13 is set/controlled by adjusting brightness in the normal observation mode. When the brightness is adjusted in the fluorescence observation mode, the opening area of the light source diaphragm 13 is fixed. In this case, the brightness is adjusted in the fluorescence observation mode by setting/controlling the amplification factor and the charge storage time.

In a first modification, an opening area of a light source diaphragm 13 is set/controlled by adjusting brightness in fluorescence observation mode. When the brightness is adjusted in normal observation mode, the opening area of the light source diaphragm 13 is fixed. In this case, the brightness is adjusted in the normal observation mode by setting/controlling an amplification factor and a charge storage time.

To be specific, as shown in Fig. 5, a comparison section 103 compares in step S8 a predetermined reference value T2 and a dimming signal P2 transmitted from a dimmer circuit 40 for a fluorescence image (see Fig. 3) on a fluorescence CCD 28a at irradiation of excitation light, and the comparison section 103 outputs a comparison result to a setting section 102.

In the case of P2 = T2, the setting section 102 sets a control circuit 37 in step S2 so as to keep an opening area S of the light source diaphragm 13 in a light source device 3 (S ← S), and then a process advances to step S9.

The setting section 102 sets an electronic shutter control section 101 so as to set and keep, at a current value, an amplification factor (gain) of an AGC 33 for the fluorescence image and a reference light image in step S9 and set and keep, at a current value, a charge storage time for the fluorescence image and the reference light image in step S10, and then the process advances to step S7.

In the case of P2 > T2, the setting section 102 sets the control circuit 37 in step S3 so as to reduce the opening area S of the light source diaphragm 13 in the light source device 3 by a predetermined amount ΔS (S ← S - ΔS), and then the process advances to step S11.

The setting section 102 sets the electronic shutter control section 101 in step S11 so as to reduce the charge storage time of the fluorescence image to a constant value, and sets the electronic shutter control section 101 in step S 12 so as to set the charge storage time of the reference light image at a constant value, and then the process advances to step S7.

In the case of P2 < T2, the setting section 102 sets the control circuit 37 in step S4 so as to increase the opening area S of the light source diaphragm 13 in the light source device 3 by the predetermined amount ΔS (S ← S + ΔS), and then the process advances to step S21.

In step S21, the setting section 102 determines whether the opening area S has reached a maximum opening area Smax or not. When the setting section 102 determines that the opening area S has reached the maximum opening area Smax, the process advances to step S13. When the setting section 102 determines that the opening area S is smaller than the maximum opening area Smax, the process advances to step S7.

In step S 13, the setting section 102 increases the amplification factor (gain) of the AGC 33 and sets the amplification factor at a constant value α2 (> a current amplification factor) for the fluorescence image. Further, in step S 14, the setting section 102 also sets the amplification factor (gain) of the AGC 33 at the constant value α2 (> the current amplification factor) for the reference light image, and the process advances to step S7.

After processing of step S7 and step S1, in the case of P1 = T1, the setting section 102 sets the electronic shutter control section 101 in step S22 so as to set and keep, at the current value, the amplification factor (gain) of the AGC 33 for a normal image and keep, at the current value, the charge storage time for controlling charge storage of an electronic shutter function of a CCD driving circuit 31 for the normal image, and then processing is completed.

After the processing of step S7 and step S1, in the case of P1 > T1, the setting section 102 sets the electronic shutter control section 101 in step S23 so as to reduce the charge storage time for controlling the charge storage of the electronic shutter function of the CCD driving circuit 31 for the normal image, and then the processing is completed.

Further, after the processing of step S7 and step S1, in the case of P1 < T1, the setting section 102 makes a setting in step S24 to increase the amplification factor (gain) of the AGC 33 for the normal image, and then the processing is completed.

In the foregoing embodiment, the opening area of the light source diaphragm 13 is set/controlled by adjusting brightness in the normal observation mode. When the brightness is adjusted in the fluorescence observation mode, the opening area of the light source diaphragm 13 is fixed. In this case, the brightness is adjusted in the fluorescence observation mode only by setting/controlling the amplification factor and the charge storage time.

Meanwhile, in the first modification, the opening area of the light source diaphragm 13 is set/controlled by adjusting brightness in the fluorescence observation mode. When the brightness is adjusted in the normal observation mode, the opening area of the light source diaphragm 13 is fixed. In this case, the brightness is adjusted in the normal observation mode only by setting/controlling the amplification factor and the charge storage time.

As described above, also in the first modification in which the brightness is adjusted first in the fluorescence observation mode and then is adjusted in the normal observation mode, the amplification factor (gain) of the AGC 33 and the charge storage time of the electronic shutter function can be set according to the observation mode with a simple configuration of the comparison section 103 and the setting section 102, thereby inexpensively adjusting the normal light image and the fluorescence image to proper brightness with ease.

Also in the present modification, even when a normal image and a fluorescence image are simultaneously obtained using the single light source device and two image pickup devices, it is possible to achieve an effect of obtaining proper brightness on both images.

### (Second modification)

In the forgoing embodiment, the electronic endoscope 2 connected to the light source device 3 and the processor 4 was described as an example. The present invention is not limited to this configuration but is also applicable to an encapsulated endoscope 200 shown in Fig. 6.

As shown in Fig. 6, the encapsulated endoscope 200 is made up of, for example, white LEDs 207a and 207b serving as light-emitting devices for irradiating a living body with illumination light, a light source section 204 serving as light-emitting device driving means for driving the white LEDs 207a and 207b, a CCD 28a for fluorescence, a CCD 28b for normal light, an excitation light cut-off filter 27, a CCD driving section 31 for driving the CCD 28a for fluorescence and the CCD 28b for normal light, a signal processing section 205 for processing image pickup signals from the CCD 28a for fluorescence and the CCD 28b for normal light, and a wireless communication section 203 for wirelessly communicating, with an outside, the signals processed in the signal processing section 205 and the light source section 204.

An AGC 33, a dimmer circuit 40, a comparison section 103, and a setting section 102 are provided in the signal processing section 205, an electronic shutter control section 101 is provided in the CCD driving section 31, and a light source control section 37A corresponding to a control part (control circuit 37) of the opening area of the light source diaphragm 13 in the foregoing embodiment is provided in the light source section 204.

As to light emitted as pulsed light from the white LEDs 207a and 207b, only light in a predetermined wavelength band is passed through spectroscopic units 208a and 208b provided on a front face of the endoscope, and the light is emitted to a living body. On the spectroscopic units 208a and 208b, the wavelength band for passage of light is selectively controlled by a spectroscopic unit control section 204a in the light source section 204. The wavelength band is switched in synchronization with the pulsed light of the white LEDs 207a and 207b, so that excitation light for exciting white light and fluorescence is emitted in time sequence.

The light source control section 37A controls driving currents of the white LEDs 207a and 207b, so that an amount of light emitted to the living body is controlled.

Also in the encapsulated endoscope configured thus, a same effect as in the foregoing embodiment or the first modification can be obtained by processing illustrated in Fig. 3 or Fig. 4. Further, in the second modification, a parameter controlled in the setting section 102 and the light source control section 37A is not "an opening area of a light source diaphragm 13" but "the driving current of the LED" and "increase (reduce) the opening area" is read as "increase (reduce) the driving current" in Fig. 3 or 4.

### (Third modification)

In the foregoing embodiment, a fluorescence observation image is picked up by the image pickup section 100a for fluorescence observation and a normal light observation image is picked up by the image pickup section 100b for normal observation. The present invention is not limited to the above image pickup. For example, as shown in Fig. 7, an endoscope apparatus 1 may be configured such that a fluorescence observation image and a normal light observation image are picked up only by a single image pickup section 100b for normal observation.

In the image pickup section 100b for normal observation of a third modification, an image pickup signal at the time of irradiation through a white light transmission filter 171 of a switching filter 17 serves as an image pickup signal of normal observation mode, and an image pickup signal at the time of irradiation through an excitation light transmission filter 172 serves as an image pickup signal of fluorescence observation mode. In the normal observation mode and the fluorescence observation mode, an opening of a diaphragm, an amplification factor, a charge storage time, and so on are controlled as in the foregoing embodiment, so that a similar operation/effect can be obtained.

### (Other modifications)

The present embodiment includes "other modifications" described below.

### Other modifications

(Modification 1) In the second modification, the light-emitting device is not limited to a white LED and may be, for example, a wavelength-tunable light-emitting device. A wavelength of illumination light is selectively controlled by the light source section 204. In this case, the spectroscopic units are not provided on a front face of the wavelength-tunable light-emitting device.
(Modification 2) Illumination light supplied from the light source device 3 to the electronic endoscope 2 or light emitted from a light-emitting device (e.g., an LED) is not limited to white light and may be time-series light of red, green, and blue.
(Modification 3) White light and excitation light may not be time-series illumination light and may be simultaneously emitted as continuous light. In this case, the excitation light is infrared light with a longer wavelength than white light, and a fluorescence image from the CCD 28a for fluorescence, a reference image, and a normal image from the CCD 28b for normal light are simultaneously obtained.
(Modification 4) Autofluorescence excited from a living tissue by excitation light was described as an example of fluorescence. The present invention is not limited to autofluorescence. Needless to say, the foregoing embodiment and the first and second modifications are also applicable to, for example, a fluorescence image obtained by fluorescence from a fluorescent agent applied to a living body. In the case of the fluorescent agent, the excitation light is, for example, near-infrared light.

The present invention is not limited to the foregoing embodiment and various changes, modifications, and so on can be made without changing the scope of the claims.

The present application is filed claiming priority based on Japanese Patent Application No. 2007-271665 which was filed on October 18, 2007.

## Claims

1. An endoscope apparatus (1) comprising:
light source means (3) configured to emit a first light which is one of normal observation light and excitation light for fluorescence observation to be irradiated to a subject and a second light which is the other of the normal observation light and the excitation light;
a light amount control means (13, 14, 17) which is provided on a light path generated from the light source means and which is configured to control an amount of light passing through the light path;
image pickup means (100a, 100b) configured to output a first image signal obtained by picking up an image of the subject irradiated with the first light whose amount is controlled by the light amount control means, and a second image pickup signal obtained by picking up an image of the subject irradiated with the second light;
dimming signal generating means (40) configured to output a first dimming signal so as to control the light amount control means in order to adjust the amount of light irradiated to the subject based on the first image pickup signal;
wherein:
the image pickup means is configured to output, as the second image pickup signal, an image pickup signal obtained by picking up an image with light from the subject irradiated with the second light whose amount of light is controlled by the light amount control means which is controlled by the first dimming signal,
the dimming signal generating means is further configured to output a second dimming signal so as to adjust brightness of the second image pickup signal based on the second image pickup signal; and
the endoscope apparatus further comprises a signal amplification means (32, 33) configured to amplify a signal intensity of the second image pickup signal picked up by the image pickup means based on the second dimming signal; or electronic shutter control means (101) configured to control an electronic shutter which is configured to store charge and to discharge the charge in the image pickup means based on the second dimming signal.

2. The endoscope apparatus according to claim 1, wherein the light amount control means is a diaphragm (13).

3. The endoscope apparatus according to claim 1, wherein the image pickup means includes
a first image pickup unit (100b) configured to pick up an image of the subject irradiated with the first light and to output the first image pickup signal, and
a second image pickup unit (100a) configured to pick up an image of the subject irradiated with the second light and to output the second image pickup signal.

4. A method of operating an endoscope apparatus (1) for adjusting brightness of an image signal picked up, the method comprising the steps of:
emitting, by light source means (3), a first light which is one of normal observation light and excitation light for fluorescence observation to be irradiated to a subject;
picking up, by image pickup means (100a, 100b), an image of the subject irradiated with the first light, and outputting a first image pickup signal;
outputting, by dimming signal generating means (40), a first dimming signal so as to adjust an amount of light irradiated to the subject based on the first image pickup signal;
controlling, based on the dimming signal, light amount control means (13, 14, 17) located on a light path from the light source means to the subject and controlling an amount of light to be irradiated to the subject;
emitting, by the light source means, the other of the normal observation light and the excitation light to be irradiated to the subject through the light amount control means being controlled;
picking up, by the image pickup means, an image of the subject irradiated with the second light through the light control means, and outputting a second image pickup signal;
outputting, by the dimming signal generating means, a second dimming signal so as to adjust a brightness of the second image pickup signal based on the second image pickup signal; and
amplifying, by signal amplification means, a signal intensity of the second image pickup signal picked up by the image pickup means based on the second dimming signal; or controlling an electronic shutter which stores charge and discharges the charge in the image pickup means based on the second dimming signal.

5. The method of operating an endoscope apparatus according to claim 4, wherein the light amount control means is a diaphragm (13).

## Patentansprüche

1. Endoskopvorrichtung (1), umfassend:
eine Lichtquelleneinrichtung (3), die konfiguriert ist, um ein erstes Licht, das eines aus einem normalen Beobachtungslicht und einem Erregungslicht für eine Fluoreszenzbeobachtung ist, das auf ein Subjekt einzustrahlen ist, und ein zweites Licht abzustrahlen, das das andere des normalen Beobachtungslichts und des Erregungslichts ist;
eine Lichtmengensteuereinrichtung (13, 14, 17), die auf einem Lichtweg vorgesehen ist, der von der Lichtquelleneinrichtung erzeugt wird, und die konfiguriert ist, um eine Menge an durch den Lichtweg passierendem Licht zu steuern;
eine Bildaufnahmeeinrichtung (100a, 100b), die konfiguriert ist, um ein erstes Bildsignal, das durch Aufnehmen eines Bildes des Subjekts, bei Beleuchtung mit dem ersten Licht, dessen Menge durch die Lichtmengensteuereinrichtung gesteuert ist, und ein zweites Bildaufnahmesignal auszugeben, das durch Aufnehmen eines Bildes des Subjekts bei Beleuchtung mit dem zweiten Licht erlangt ist;
eine Abdunkelungssignalerzeugungseinrichtung (40), die konfiguriert ist, um ein erstes Abdunkelungssignal auszugeben, um die Lichtmengensteuereinrichtung zu steuern, um wiederum die Menge an zu dem Subjekt abgestrahltem Licht auf der Grundlage des ersten Bildaufnahmesignals einzustellen;
wobei:
die Bildaufnahmeeinrichtung konfiguriert ist, um als das zweite Bildaufnahmesignal ein Bildaufnahmesignal auszugeben, das erlangt ist, indem ein Bild mit Licht von dem mit dem zweiten Licht bestrahlten Subjekt aufgenommen wird, dessen Menge an Licht durch die Lichtmengensteuereinrichtung gesteuert wird, die wiederum durch das erste Abdunkelungssignal gesteuert wird,
wobei die Abdunkelungssignalerzeugungseinrichtung weiterhin konfiguriert ist, um ein zweites Abdunkelungssignal abzugeben, um eine Helligkeit des zweiten Bildaufnahmesignals auf der Grundlage des zweiten Bildaufnahmesignals einzustellen; und
die Endoskopvorrichtung weiterhin eine Signalverstärkungseinrichtung (32, 33) umfasst, die konfiguriert ist, um eine Signalintensität des zweiten Bildaufnahmesignals, das durch die Bildaufnahmeeinrichtung aufgenommen ist, auf der Grundlage des zweiten Abdunkelungssignals zu verstärken; oder eine elektronische Shuttersteuereinrichtung (101), die konfiguriert ist, um einen elektronischen Shutter zu steuern, der konfiguriert ist, um eine Ladung zu speichern und die Ladung in die Bildaufnahmeeinrichtung auf der Grundlage des zweiten Abdunkelungssignals zu entladen.

2. Endoskopvorrichtung gemäß Anspruch 1, wobei die Lichtmengensteuereinrichtung eine Fotoblende (13) ist.

3. Endoskopvorrichtung gemäß Anspruch 1, wobei die Bildaufnahmeeinrichtung umfasst
eine erste Bildaufnahmeeinheit (100b), die konfiguriert ist, um ein Bild des mit dem ersten Licht bestrahlten Subjekts aufzunehmen und das erste Bildaufnahmesignal auszugeben, und
eine zweite Bildaufnahmeeinheit (100a), die konfiguriert ist, um ein Bild des mit dem zweiten Licht bestrahlen Subjekts aufzunehmen und das zweite Bildaufnahmesignal auszugeben.

4. Verfahren zum Betreiben einer Endoskopvorrichtung (1) zum Einstellen einer Helligkeit eines aufgenommenen Bildsignals, wobei das Verfahren die Schritte umfasst:
Abstrahlen, durch eine Lichtquelleneinrichtung (3), eines ersten Lichts, das eines aus einem normalen Beobachtungslicht und einem Erregungslicht für eine Fluoreszenzbeobachtung ist, das auf ein Subjekt einzustrahlen ist;
Aufnehmen, durch eine Bildaufnahmeeinrichtung (100a, 100b), des Subjekts, das mit dem ersten Licht bestrahlt ist, und Ausgeben eines ersten Bildaufnahmesignals;
Ausgeben, durch eine Abdunkelungssignalerzeugungseinrichtung (40), eines ersten Abdunkelungssignals, um eine Menge an Licht, die auf das Subjekt einstrahlt, auf der Grundlage des ersten Bildaufnahmesignals einzustellen;
Steuern, auf der Grundlage des Abdunkelungssignals, einer Lichtmengensteuereinrichtung (13, 14, 17), die sich auf einem Lichtweg von der Lichtquelleneinrichtung zu dem Subjekt befindet, und Steuern einer Menge an Licht, die auf das Subjekt einzustrahlen ist;
Abstrahlen, durch die Lichtquelleneinrichtung, des anderen des normalen Beobachtungslichts und des Erregungslichts, das auf das Subjekt einzustrahlen ist, durch die Lichtmengensteuereinrichtung und deren Steuerung;
Aufnehmen, durch die Bildaufnahmeeinrichtung, eines Bildes des mit dem zweiten Licht bestrahlten Subjekts durch die Lichtsteuereinrichtung, und Ausgeben eines zweiten Bildaufnahmesignals;
Ausgeben, durch die Abdunkelungssignalerzeugungseinrichtung, eines zweiten Abdunkelungssignals, um eine Helligkeit des zweiten Bildaufnahmesignals auf der Grundlage des zweiten Bildaufnahmesignals einzustellen; und
Verstärken, durch eine Signalverstärkungseinrichtung, einer Signalintensität des zweiten Bildaufnahmesignals, das durch die Bildaufnahmeeinrichtung aufgenommen ist, auf der Grundlage des zweiten Abdunkelungssignals; oder Steuern eines elektronischen Shutters, der eine Ladung speichert und die Ladung in die Bildaufnahmeeinrichtung auf der Grundlage des zweiten Abdunkelungssignals entlädt.

5. Verfahren zum Betreiben einer Endoskopvorrichtung gemäß Anspruch 4, wobei die Lichtmengensteuereinrichtung eine Fotoblende (13) ist.

## Revendications

1. Appareil (1) d'endoscope comprenant :
un moyen (3) de source de lumière configuré pour émettre une première lumière qui est l'une parmi une lumière d'observation normale et une lumière d'excitation pour une observation en fluorescence devant être irradiée vers un sujet et une deuxième lumière qui est l'autre parmi la lumière d'observation normale et la lumière d'excitation ;
un moyen (13, 14, 17) de commande de quantité de lumière qui est prévu sur un trajet de lumière généré à partir du moyen de source de lumière et qui est configuré pour commander une quantité de lumière passant à travers le trajet de lumière ;
un moyen (100a, 100b) de capture d'image configuré pour délivrer en sortie un premier signal d'image obtenu en capturant une image du sujet irradié avec la première lumière dont la quantité est commandée par le moyen de commande de quantité de lumière, et un deuxième signal de capture d'image obtenu en capturant une image du sujet irradié avec la deuxième lumière ;
un moyen (40) de génération de signal de gradation configuré pour délivrer en sortie un premier signal de gradation de façon à commander le moyen de commande de quantité de lumière afin d'ajuster la quantité de lumière irradiée vers le sujet sur la base du premier signal de capture d'image ;
dans lequel :
le moyen de capture d'image est configuré pour délivrer en sortie, en tant que deuxième signal de capture d'image, un signal de capture d'image obtenu en capturant une image avec la lumière provenant du sujet irradié avec la deuxième lumière dont la quantité de lumière est commandée par le moyen de commande de quantité de lumière qui est commandé par le premier signal de gradation,
le moyen de génération de signal de gradation est en outre configuré pour délivrer en sortie un deuxième signal de gradation de façon à ajuster la luminosité du deuxième signal de capture d'image sur la base du deuxième signal de capture d'image ; et
l'appareil d'endoscope comprend en outre un moyen (32, 33) d'amplification de signal configuré pour amplifier une intensité de signal du deuxième signal de capture d'image capturée par le moyen de capture d'image sur la base du deuxième signal de gradation ; ou un moyen (101) de commande d'obturateur électronique configuré pour commander un obturateur électronique qui est configuré pour stocker la charge ou pour évacuer la charge dans le moyen de capture d'image sur la base du deuxième signal de gradation.

2. Appareil d'endoscope selon la revendication 1, dans lequel le moyen de commande de quantité de lumière est un diaphragme (13).

3. Appareil d'endoscope selon la revendication 1, dans lequel le moyen de capture d'image comprend
une première unité (100b) de capture d'image configurée pour capturer une image du sujet irradié avec la première lumière et pour délivrer en sortie le premier signal de capture d'image, et
une deuxième unité (100a) de capture d'image configurée pour capturer une image du sujet irradié avec la deuxième lumière et pour délivrer en sortie le deuxième signal de capture d'image.

4. Procédé de fonctionnement d'un appareil (1) d'endoscope destiné à ajuster la luminosité d'un signal d'image capturée, le procédé comprenant les étapes consistant à :
émettre, par un moyen (3) de source de lumière, une première lumière qui est l'une parmi une lumière d'observation normale et une lumière d'excitation pour une observation en fluorescence devant être irradiée vers un sujet ;
capturer, par un moyen (100a, 100b) de capture d'image, une image du sujet irradié avec la première lumière, et délivrer en sortie un premier signal de capture d'image ;
délivrer en sortie, par un moyen (40) de génération de signal de gradation, un premier signal de gradation de façon à ajuster une quantité de lumière irradiée vers le sujet sur la base du premier signal de capture d'image ;
commander, sur la base du signal de gradation, un moyen (13, 14, 17) de commande de quantité de lumière placé sur un trajet de lumière allant du moyen de source de lumière au sujet et commander une quantité de lumière devant être irradiée vers le sujet ;
émettre, par le moyen de source de lumière, l'autre parmi la lumière d'observation normale et la lumière d'excitation devant être irradiée vers le sujet à travers le moyen de commande de quantité de lumière étant commandé ;
capturer, par le moyen de capture d'image, une image du sujet irradié avec la deuxième lumière à travers le moyen de commande de lumière, et délivrer en sortie un deuxième signal de capture d'image ;
délivrer en sortie, par le moyen de génération de signal de gradation, un deuxième signal de gradation de façon à ajuster une luminosité du deuxième signal de capture d'image sur la base du deuxième signal de capture d'image ; et
amplifier, par un moyen d'amplification de signal, une intensité de signal du deuxième signal de capture d'image capturée par le moyen de capture d'image sur la base du deuxième signal de gradation ; ou commander un obturateur électronique qui stocke la charge et décharge la charge dans le moyen de capture d'image sur la base du deuxième signal de gradation.

5. Procédé de fonctionnement d'un appareil d'endoscope selon la revendication 4, dans lequel le moyen de commande de quantité de lumière est un diaphragme (13).
